# FASCICULE DE BREVET EUROPEEN

(11) **EP 2 807 144 B1**
(45) Date de publication et mention de la délivrance du brevet: **27.03.2019**
(21) Numéro de dépôt: 13704179.4
(22) Date de dépôt: 18.01.2013
(51) Int. Cl.: C07C 231/24, C07C 233/09

(54) **NOUVEAU PROCÉDÉ DE CONCENTRATION DE SPILANTHOL ET/OU DE SES ISOMÈRES ET/OU HOMOLOGUES**
NEUES VERFAHREN ZUM KONZENTRIEREN VON SPILANTHOL UND/ODER ISOMEREN UND/ODER HOMOLOGEN DAVON
NOVEL PROCESS FOR CONCENTRATING SPILANTHOL AND/OR ISOMERS AND/OR HOMOLOGUES THEREOF

(30) Priorité: 24.01.2012 FR 1250657
(43) Date de publication de la demande: 03.12.2014
(73) Titulaire: Robertet S.A., 06130 Grasse (FR)
(72) Inventeur: CASAZZA, André, F-06130 Grasse (FR); KORMANN, Karine, F-06560 Valbonne (FR)
(74) Mandataire: Santarelli
(86) Numéro de dépôt international: PCT/FR2013/050121
(87) Numéro de publication internationale: WO 2013/110883

(56) Documents cités:
- JP-A- 7 090 294
- US-A1- 2008 171 003
- MARTIN JACOBSON: JOURNAL OF THE AMERICAN CHEMICAL SOCIETY, vol. 78, no. 19, 5 octobre 1956 (1956-10-05), pages 5084-5087, XP055037747, ISSN: 0002-7863, DOI: 10.1021/ja01600a071
- ICHIRO YASUDA, KOICHI TAKEYA, HIDEJI ITOKAWA: "The geometric structure of spilanthol", CHEMICAL AND PHARMACEUTICAL BULLETIN, vol. 28, no. 7, 1 mars 1980 (1980-03-01), pages 2251-2253, XP002683240,
- FRED ACREE ET AL: THE JOURNAL OF ORGANIC CHEMISTRY, vol. 10, no. 3, 1 mai 1945 (1945-05-01), pages 236-242, XP055037794, ISSN: 0022-3263, DOI: 10.1021/jo01179a014

## Description

L'invention se place dans le domaine de la chimie, particulièrement dans le domaine des procédés de concentration d'un produit contenu dans une composition. Plus particulièrement l'invention vise un nouveau procédé de concentration de composés amides et très particulièrement du spilanthol.

Les extraits de Jambu (teintures, concrètes, absolus, Huile essentielle, oléorésines, distillats, etc.) peuvent être obtenus de toutes les parties des plantes du genre Spilanthes de la famille des Compositae (Spilanthes acmella or acmelia, Spilanthes ciliata, Spilanthes acmella var. Murr., Spilanthes acmella var. oleracea, Spilanthes americana, Spilanthes nervosa, Spilanthes leiocarpa, Spilanthes paraguyensis, Spilanthes chamaecaula, Spilanthes macraei, Spilanthes oleracea, Spilanthes oppositifolia, Spilanthes alba, Spilanthes calvus., Spilanthes mauritiana, Spilanthes costata Spilanthes urens, Spilanthes anactina, Spilanthes repens, Spilanthes beccabunga, préférentiellement de Spilanthes acmella var. oleracea (encore dénommée Spilanthes acmella oleracea ou Acmella oleracea).

Les inflorescences des plantes du genre Spilanthes, composées de fleurs jaunes, et les feuilles ont une saveur piquante. Dans l'État brésilien du Para, les plantes et/ou les extraits sont un élément fondamental de la cuisine traditionnelle ainsi qu'en médecine traditionnelle.

Les propriétés de ces végétaux sont attribuées principalement au spilanthol, également appelé affinine, ou (2E,6Z,8E)-N-(2-methylpropyl)deca-2,6,8-trienamide [CAS 25394-57-4] (dénommé par ailleurs dans le présent texte "spilanthol vrai"), qui est une isobutylamide ayant la formule suivante :

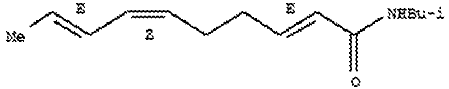

De par sa structure ce composé peut présenter de nombreux isomères.

Il est par ailleurs connu (Martin R. et Becker H., Phytochemistry, vol. 23, No 8, pp. 1781-1783, 1984) que les extraits de végétaux comprenant du spilanthol vrai, comprennent en outre des homologues dudit spilanthol vrai parmi lesquels par exemple ceux qui répondent à la formule I suivante

R2-CH2-CH=CH-CH=CH-CH2-CH2-CHA-CHA-CO-NH-R1

dans laquelle
R1 peut représenter un radical -(CH2)-CH(CH3)2, ou un radical -(CH2)-CH(CH3)-CH2-CH3, ou un radical -CH2-CH2-C6H5 ; et
R2 peut représenter un atome d'hydrogène ou un radical -O-CO-CH2-CH(CH3)2, ou un radical -O-CO-CH=CH(CH3)2 et
A représente un atome d'hydrogène ou pris ensemble avec les atomes de carbone qui les portent forment une double liaison.

A titre d'exemple on peut citer comme homologues du spilanthol vrai l'acide 2-méthylbutylamide spilantique (R1 = -(CH2)-CH(CH3)-CH2-CH3, R2 = H, et A forment une double liaison), l'acide 2-phénylethylamide spilantique (R1 = -CH2-CH2-C6H5, R2 = H et A forment une double liaison), le 10-hydroxyspilantholisovalerate (R1 = -(CH2)-CH(CH3)2, R2 = -O-CO-CH2-CH(CH3)2 et A forment une double liaison), le 10-hydroxyspilanthol-(3-methylacrylate) (R1 = -(CH2)-CH(CH3)2, R2 = -O-CO-CH=CH(CH3)2 et A forment une double liaison), ou encore l'hydrospilanthol ou 2,3-dihydrospilanthol (R1 = -(CH2)-CH(CH3)2, R2 et A = H).

En règle générale dans les extraits végétaux qui en contiennent le (2E,6Z,8E)-N-(2-methylpropyl)deca-2,6,8-trienamide (par ailleurs appelé dans le présent texte "spilanthol vrai") est quantitativement en très large majorité par rapport à ces homologues.

Ainsi dans le présent texte l'emploi du terme "spilanthol" couvrira, sauf indication contraire, aussi bien le spilanthol vrai ((2E,6Z,8E)-N-(2-methylpropyl)deca-2,6,8-trienamide) isolé, que les mélanges du spilanthol vrai avec ses isomères et/ou homologues qui peuvent l'accompagner.

Le spilanthol est connu pour avoir de nombreuses applications.

Il est utilisé, par exemple en médecine comme analgésique pour les maux de dents, pour le traitement des aphtes et l'herpès, pour la stomatite et les infections de la gorge, comme un sialagogue ou encore comme un cicatrisant.

Des activités insecticides ont été décrites comme en particulier larvicide contre les larves de Culex quinquefasciatus, contre Aedes aegyptii, contre les tiques, les cafards ou encore les mites.

Le spilanthol a aussi été décrit comme agent antimicrobien efficace particulièrement contre Proteus mirabilis, Pseudomonas aeruginosa, Staphylococcus aureus ou Candida albicans, comme fongistatique et fongicide par exemple contre Aspergillus spp, ou encore comme agent antimutagène.

Le spilanthol entre également dans la fabrication de compositions cosmétiques antirides ou dans des compositions cosmétiques pour les peaux stressées.

D'autres propriétés du spilanthol sont également largement utilisées comme par exemple son aptitude à procurer une sensation de picotement par exemple pour la prolongation de la sensation gazeuse particulière aux boissons gazeuses, ou rafraichissante dans des compositions cosmétiques. On le retrouve également dans de nombreuses compositions de bonbons ou de chewing-gums.

Le spilanthol est donc un composé largement utilisé.

Il est notable qu'outre l'oléorésine de Jambu, d'autres extraits d'autres plantes sont connus pour comprendre du spilanthol. On citera à cet égard les plantes du genre Heliopsis de la famille des Compositae, par exemple les espèces Heliopsis buphthalmoides, Heliopsis gracilis, Heliopsis helianthoides, Heliopsis longipes, Heliopsis oppositifolia, ou encore Heliopsis parvifolia.

La préparation du spilanthol peut être soit purement synthétique (par exemple comme celle décrite dans WO/2011/007807) ou encore par extraction à partir de Jambu comme il est par exemple décrit dans la demande de brevet US2008/0171003.

Le document JP7090294 décrit l'extraction de spilanthol à partir de *Spilanthes oleracea Jacq.* ou de *spilanthes acmella var. boninensis f. fusca* à l'aide d'un solvant polaire et d'un solvant non polaire non miscibles l'un avec l'autre. Par ce procédé il est obtenu à partir d'une huile essentielle contenant de 10 à 40% en poids de spilanthol, une huile essentielle comprenant plus de 50% en poids de spilanthol.

Le document Ichiro Yasuda et col. (Chemical and pharmaceutical Bulletin, vol.28, no.7, 1er mars 1980, pages 2251-2253) décrit l'isolation du spilanthol à partir de *Spilanthes oleracea* et divulgue la structure de ce composé. L'extraction est réalisée avec du méthanol, puis de l'hexane. L'extrait obtenu est appliqué sur une colonne de silice pour être purifié.

Mais de manière générale il est difficile d'obtenir facilement, industriellement et à un coût le plus bas possible des compositions fortement concentrées en spilanthol voire des compositions ne contenant que 100% de spilanthol au sens du terme défini précédemment.

Ainsi il demeure un besoin en méthode de production du spilanthol qui surmonte les inconvénients (difficultés, complexités, longueur, coût, mauvais rendement, etc.) connus des méthodes décrites dans l'art antérieur (sans bien entendu remettre en cause les qualités de celles-ci).

L'invention vise donc un nouveau procédé de concentration du spilanthol, (spilanthol vrai, ses isomères et homologues de type amide comme par exemple ceux répondant à la formule I), simple, rapide, peu coûteux et facilement industrialisable qui permet à partir de toute composition comprenant du spilanthol à n'importe quelle concentration, d'obtenir une composition comprenant 100% d'un ensemble constitué de spilanthol vrai et éventuellement des isomères et/ou des homologues amides dudit spilanthol vrai, ladite composition comprenant au moins 90 % de spilanthol vrai, avantageusement 95%.

Selon l'invention, ladite composition obtenue en fin de procédé peut être sous toute forme envisageable, mais généralement se présente sous la forme d'un liquide épais huileux. Toute méthode permettant de déterminer la nature des composés contenus dans ladite composition peut être utilisée. On citera à titre d'exemple la technique de
chromatographie en phase liquide haute performance (HPLC) ou celle de chromatographie en phase gazeuse (GC) avec étalonnage interne selon les techniques classiques couramment utilisée en chimie, comme par exemple décrit dans Chemical Analysis, Modem Instrumental Methods and Techniques (Francis & Annick Rouessac, 2000, John Wiley & Sons, Ltd, editors). L'invention vise un nouveau procédé de concentration du spilanthol, contenu dans une composition, comprenant la formation d'un dérivé d'addition du spilanthol, ainsi éventuellement que de ses isomères et homologues de type amide, avec un acide fort (étape d'addition), dérivé d'addition qui peut être ensuite hydrolysé pour régénérer ledit spilanthol, ainsi éventuellement que ses isomères et homologues de type amide, sous la forme d'une composition comprenant du spilanthol vrai, ainsi éventuellement que ses isomères et homologues de type amide.

Ainsi l'invention a pour objet un procédé de concentration du spilanthol contenu dans une composition, comprenant:
a. une première étape de mélange d'une composition comprenant du spilanthol avec un solvant inerte aux acides choisi parmi un éther choisi parmi le Diéthyléther, le di-isopropyléther, le Méthyl tert-butyl éther, le Tétrahydrofuranne, le Méthyltétrahydrofuranne, le Dioxane, le Dimethoxyethane, l'Anisole, les éthers de Crown ou encore le Polyethylene glycol, un hydrocarbure choisi parmi les alcanes linéaires ou ramifiés, substitués ou non, comprenant entre 5 et 8 atomes de carbone, ou les cycloalcanes et composés aromatiques substitués ou non, comprenant entre 5 et 8 atomes de carbone ou une cétone choisie parmi l'acétone, la méthyléthylcétone, la méthylisobutylcétone, la pinacolone, la diéthylcétone, la diisopropylcétone, ou encore la 2,4 diméthyl 3 pentanone pour obtenir une solution anhydre comprenant du spilanthol;
b. une seconde étape de formation d'un produit huileux comprenant un dérivé d'addition du spilanthol par mélange de la solution anhydre obtenue à la première étape avec un acide fort choisi parmi l'acide chlorhydrique, l'acide sulfurique concentré, les acides phosphoriques, l'acide oxalique, l'acide nitrique, l'acide iodhydrique, l'acide bromhydrique, l'acide perchlorique, l'acide chlorique, l'acide permanganique, l'acide manganique, l'acide fluoroantimonique, l'acide magique, l'acide méthanesulfonique, l'acide trifluorométhanesulfonique ou acide triflique, l'acide fluorosulfurique ou acide fluorosulfonique, l'acide naphtalène-1-sulfonique, l'acide naphtalène-1,5-disulfonique, l'acide disulfurique ou oléum, ou encore l'acide paratoluènesulfonique;
c. une troisième étape d'hydrolyse dudit composé d'addition compris dans le produit huileux obtenu à la seconde étape, par mise en présence d'une base choisie parmi le carbonate de sodium, le bicarbonate de sodium, la chaux, l'ammoniaque, la soude, la lithine, la potasse, ou encore la baryte pour obtenir une solution de spilanthol.

Selon l'invention, ladite composition comprenant du spilanthol (au sens défini précédemment à savoir spilanthol vrai, isomères et homologues de type amide), peut être toute composition qu'elle soit naturelle ou synthétique qui contient du spilanthol en une quantité quelconque que l'on veut concentrer.

Par composition naturelle comprenant du spilanthol on entend une composition obtenue à partir d'un produit naturel comme par exemple une plante, parmi lesquelles on citera celles de la famille des Compositae, avantageusement celles des genres Spilanthes et Heliopsis.

A cet égard les compositions naturelles comprenant du spilanthol peuvent être tout type d'extrait quel que soit le mode opératoire dont il résulte (extraction, distillation). On citera à cet égard les extraits aqueux ou les extraits non aqueux, particulièrement ceux obtenus à l'aide de solvants. On peut citer les oléorésines, les huiles essentielles, l'absolue, les concrètes ou encore les extraits par Hydrofluorocarbone (HFC).

Selon l'invention, les compositions naturelles comprenant du spilanthol peuvent être tout type d'extrait de toutes les parties des plantes considérées.

Par composition synthétique comprenant du spilanthol on entend toute composition comprenant du spilanthol, obtenue autrement que par extraction ou distillation à partir d'un produit naturel, comme par exemple après une synthèse chimique ou une hémi-synthèse à partir d'un précurseur naturel.

Les acides forts utilisables selon l'invention sont l'acide chlorhydrique (HCI), l'acide sulfurique concentré (H2SO4 à 92-98%), les acides phosphoriques, particulièrement l'acide orthophosphorique (H3PO4), l'acide oxalique, particulièrement l'acide oxalique anhydre (C2H2O4), l'acide nitrique (HNO3), l'acide iodhydrique (HI), l'acide bromhydrique (HBr), l'acide perchlorique (HClO4), l'acide chlorique (HClO3), l'acide permanganique (HMnO4), l'acide manganique (H2MnO4), l'acide fluoroantimonique (HF·SbF5), l'acide magique (HSO3F·SbF5), l'acide méthanesulfonique (CH3SO3H), l'acide trifluorométhanesulfonique ou acide triflique (HSO3CF3), l'acide fluorosulfurique ou acide fluorosulfonique (HSO3F), l'acide naphtalène-1-sulfonique (C10H7SO3H), l'acide naphtalène-1,5-disulfonique (C10H6(SO3H)2), l'acide disulfurique ou oléum (H2S2O7), ou encore l'acide paratoluènesulfonique (APTS).

Préférentiellement, selon l'invention on pourra utiliser un acide fort choisi parmi l'acide sulfurique concentré (H2SO4), les acides phosphoriques, particulièrement l'acide orthophosphorique (H3PO4), l'acide oxalique, particulièrement l'acide oxalique anhydre (C2H2O4), très préférentiellement, l'acide sulfurique concentré.

On comprend donc qu'à la deuxième étape du procédé selon l'invention, un dérivé d'addition du spilanthol contenu dans une composition a été obtenu par acidification de ladite composition. Ce dérivé d'addition correspond à un complexe d'addition de l'acide fort sur la fonction basique de l'amide. La troisième étape du procédé selon l'invention va permettre la régénération du spilanthol. Cette étape est réalisée par addition d'une base au milieu comprenant le dérivé d'addition obtenu à la première étape.

Selon l'invention, la base utilisable à la seconde étape d'hydrolyse est du carbonate de sodium, du bicarbonate de sodium, de la chaux (Ca(OH)2), de l'ammoniaque, de la soude, de la lithine (LiOH), de la potasse (KOH), ou encore de la baryte (Ba(OH)2). Préférentiellement selon l'invention la base utilisable à la seconde étape d'hydrolyse peut être du carbonate de sodium ou du bicarbonate de sodium.

Selon l'invention, la première étape du procédé d'isolation du spilanthol est réalisée en milieu anhydre. On entend ici qu'à la première étape du procédé qu'au moins la composition comprenant le spilanthol est mélangée à un solvant inerte aux acides et que éventuellement l'acide fort utilisé est également mélangé à un solvant inerte aux acides.

Un autre avantage de réaliser la première étape du procédé d'isolation du spilanthol en milieu anhydre est l'obtention d'un complexe insoluble aisément séparable du milieu réactionnel par simple décantation.

En général, les réactifs "purs" du commerce tels que les acides forts listés précédemment conviennent parfaitement sans avoir à les soumettre à traitement desséchant. Mais si cela s'avère nécessaire ces réactifs peuvent être mélangés avant réaction avec au moins un solvant inerte aux acides, permettant leur dessiccation. Il peut donc être nécessaire selon l'invention que les différents réactifs intervenant dans la première étape du procédé soient anhydres ou considérés comme tels. Très avantageusement, les différents réactifs intervenant dans la première étape du procédé peuvent ne pas comprendre plus de 0,5% d'eau.

Selon l'invention, la solution de spilanthol obtenue à la fin du procédé peut être utilisée immédiatement ou subir toutes opérations nécessaires à l'élimination des éventuels résidus de solvant inerte aux acides qu'elle peut encore comporter, à son séchage, à sa filtration, à son changement d'aspect.

Selon l'invention, lesdits solvants inertes aux acides utilisables sont choisis parmi les éthers choisis parmi le Diéthyléther, le di-isopropyléther, le Méthyl tert-butyl éther (MTBE), le Tétrahydrofuranne (THF), le Méthyltétrahydrofuranne (MeTHF), le Dioxane, le Dimethoxyethane (DME), l'Anisole (methoxybenzene), les éthers de Crown ou encore le Polyethylene glycol (PEG),
les hydrocarbures choisis parmi les alcanes linéaires ou ramifiés, substitués ou non, comprenant entre 5 et 8 atomes de carbone, comme par exemple le n-pentane, l'isopentane, le néopentane, le n-hexane, le 2-méthylpentane, le 3-méthylpentane, le 2,2-diméthylbutane, le 2,3-diméthylbutane, le n-heptane, le 2-méthylhexane, le 3-méthylhexane ou son mélange racémique de R-3-méthylhexane et S-3-méthylhexane, le 2,2-diméthylpentane, le 2,3-diméthylpentane ou son mélange racémique de R-2,3-diméthylpentane et S-2,3-diméthylpentane, le 2,4-diméthylpentane, le 3,3-diméthylpentane, le 3-éthylpentane, le 2,2,3-triméthylbutane, le n-octane, le 2-méthylheptane, le 3-méthylheptane ou son mélange racémique de (R)-3-méthylheptane et (S)-3-méthylheptane, le 4-méthylheptane, le 3-éthylhexane, le 2,2-diméthylhexane, le 2,3-diméthylhexane ou son mélange racémique de (R)-2,3-diméthylhexane et (S)-2,3-diméthylhexane, le 2,4-diméthylhexane ou son mélange racémique de (R)-2,4-diméthylhexane et (S)-2,4-diméthylhexane, le 2,5-diméthylhexane, le 3,3-diméthylhexane, le 3,4-diméthylhexane ou son mélange racémique et ses diastéréoisomères (composé méso) (R,R)-3,4-diméthylhexane, (S,S)-3,4-diméthylhexane, (3R,4S)-3,4-diméthylhexane ou (3S,4R)-3,4-diméthylhexane, le 3-éthyl-2-méthylpentane, le 3-éthyl-3-méthylpentane, le 2,2,3-triméthylpentane ou son mélange racémique de (R)-2,2,3-triméthylpentane et (S)-2,2,3-triméthylpentane, le 2,2,4-triméthylpentane ou isooctane, le 2,3,3-triméthylpentane, le 2,3,4-triméthylpentane, ou encore le 2,2,3,3-tétraméthylbutane ;
les cycloalcanes et composés aromatiques substitués ou non, comprenant entre 5 et 8 atomes de carbone, comme par exemple le cyclopentane, le cyclohexane, le cycloheptane, le cyclooctane, le xylène, le benzene, ou encore le toluene, préférentiellement le benzene , et leurs dérivés ou isomères ;
les cétones choisies parmi l'acétone, la méthyléthylcétone, la méthylisobutylcétone (MIBK), la pinacolone, la diéthylcétone (3 pentanone), la diisopropylcétone, ou encore la 2,4 diméthyl 3 pentanone, préférentiellement méthylisobutylcétone.

Préférentiellement selon l'invention on pourra utiliser le Diéthyléther, le di-isopropyléther, le Méthyl tert-butyl ether (MTBE), le Méthyltétrahydrofuranne (MeTHF), le benzène, le toluène ou encore la méthylisobutylcétone (MIBK), très préférentiellement le diisopropyléther.

Selon l'invention, l'étape de mise en solution anhydre d'une composition comprenant du spilanthol, peut être réalisée par mélange de ladite composition comprenant du spilanthol avec ledit solvant inerte aux acides pour obtenir une solution finale comprenant ladite composition comprenant du spilanthol en une proportion comprise entre 20 et 50 % (poids/volume), préférentiellement en une proportion comprise entre 30 et 40 % (poids/volume).

Selon l'invention, lorsqu'à l'étape d'addition l'acide fort utilisé est lui-même en solution en milieu anhydre, ladite solution anhydre d'acide fort peut être obtenue par mélange dudit acide fort avec ledit solvant inerte aux acides pour obtenir une solution finale comprenant de l'acide fort en une proportion comprise entre 2 et 15 % (volume/volume), préférentiellement en une proportion comprise entre 3 et 14% (volume/volume).

Selon l'invention, à l'étape d'hydrolyse (ou de régénération), ladite base peut être ajoutée audit produit huileux obtenu à la seconde étape en un volume représentant de 25% à 75% du volume dudit produit huileux, avantageusement représentant de 40 à 60 % du volume dudit produit huileux.

Selon l'invention ladite base utilisée à l'étape d'hydrolyse (ou de régénération) peut être en solution, avantageusement aqueuse, en une proportion comprise entre 5 et 15 % avantageusement 10%.

Selon l'invention, l'étape d'addition par mélange de ladite solution comprenant du spilanthol, anhydre, avec un acide fort peut être réalisée par incorporation de l'une des solutions dans l'autre, avantageusement de la solution acide dans la solution comprenant du spilanthol, sous agitation, à température ambiante (environ 25°C +/- 5°C). Lorsque les solutions sont mélangées, ledit mélange peut être maintenu sous agitation pendant un temps compris entre 1 et 20 minutes, avantageusement entre 1 et 5 minutes. A la fin de l'agitation on laisse ledit produit huileux formé décanter avant de le récupérer pour réaliser l'étape de régénération permettant de récupérer le spilanthol qu'elle comprend.

L'étape d'hydrolyse (ou de régénération) peut être réalisée par mélange dudit produit huileux avec la base sous agitation pendant un temps compris entre 5 et 120 mn, avantageusement entre 5 et 30 mn. Il est possible au cours de cette phase du procédé de vérifier la consommation complète de l'acide par une mesure du pH de la solution obtenue, qui lorsqu'il est compris entre 7 et 10, avantageusement entre 7,5 et 8,5 indique que l'acide a été totalement neutralisé.

On laisse alors décanter la solution et on récupère la phase organique qui peut alors subir toutes opérations nécessaires à l'élimination des résidus dudit solvant inerte aux acides qu'elle peut encore comporter, à son séchage, à sa filtration, à sa concentration. Ainsi il est possible par exemple de procéder à une étape de lavage de la phase organique. Cette étape est facultative, l'homme du métier saura réaliser un lavage en cas de besoin. La solution obtenue peut comprendre 100% soit de spilanthol vrai ((2E,6Z,8E)-N-(2-methylpropyl)deca-2,6,8-trienamide) isolé, soit d'un mélange de spilanthol vrai avec ses isomères et/ou homologues qui peuvent l'accompagner.

Selon l'acide fort utilisé, il est possible qu'à l'étape de formation d'un dérivé d'addition du spilanthol avec un acide fort, après mélange de la composition comprenant le spilanthol avec l'acide fort, la solution obtenue ne se présente pas sous la forme d'insoluble. Il peut alors être nécessaire de faire subir à cette solution toutes étapes nécessaires afin que deux phases se séparent. L'Homme du métier n'aura aucun problème à réaliser ces étapes supplémentaires parfaitement habituelles en synthèse chimique. Il pourra par exemple évaporer ledit solvant inerte aux acides résiduels jusqu'à séparation des phases, par exemple en utilisant un évaporateur rotatif. Le résidu alors obtenu pourra alors être repris et lavé dans un solvant comme par exemple l'hexane, le cyclohexane ou encore le diisopropyléther avant d'être traité avec ladite base afin de réaliser l'étape de régénération.

Il est notable que pour tous les exemples décrits ci-après les chromatographies réalisées pour s'assurer de la nature des composés contenus dans le produit huileux obtenu en fin de procédé sont tous superposables et ne présentent que des pics correspondants au spilanthol vrai, largement majoritaire, et à ces homologues.

### Exemples

### Exemple 1 : concentration du spilanthol contenu dans un extrait de Jambu

55,0 g d'absolue de Jambu titrée à 35% de spilanthol vrai sont mis en solution dans 165 mL d'éther éthylique. En même temps 8,0 mL d'acide sulfurique concentré sont dissous dans 240 mL d'éther éthylique.

Sous bonne agitation, la solution d'acide est versée sur la solution de Jambu. Un changement de coloration apparait. Le mélange est maintenu sous agitation encore pendant une ou deux minutes et puis laissé à décanter. Rapidement, une phase huileuse noire se rassemble au fond du récipient.

Cette phase huileuse noire est alors soutirée et elle est agitée en présence de 100 mL d'éther éthylique, puis de nouveau laissée à décanter.

La phase huileuse noire est reprise par 300 mL d'éther éthylique et agitée en présence de 175 mL de carbonate de sodium à 10% dans l'eau.

La mesure du pH permet de vérifier que tout l'acide a bien été neutralisé (pH attendu 8,0) et la décantation poursuivie.

La solution éthérée contenant maintenant le spilanthol est alors séchée, filtrée et concentrée à l'évaporateur rotatif pour fournir 21,0 g d'une huile comprenant 100% d'un mélange à au moins 90% de spilanthol vrai accompagné de ses isomères et homologues amides.

### Exemple 2 : concentration du spilanthol contenu dans un extrait de Jambu

60,0 g d'oléorésine de Jambu titrée à 60% de spilanthol vrai sont mis en solution dans 180 mL de diisopropyl éther. En même temps 9,0 mL d'acide sulfurique concentré sont mis en solution dans 225 mL de diisopropyl éther.

La réaction est réalisée exactement de la même manière que pour l'exemple 1, pour obtenir 39,0 g d'une huile comprenant 100% d'un mélange à au moins 90% de spilanthol vrai accompagné de ses isomères et homologues amides.

### Exemple 3 : concentration du spilanthol contenu dans un extrait de Jambu

60,0 g d'oléorésine Jambu titrée à 60% de spilanthol vrai sont mis en solution dans 180 mL de diisopropyl éther et 17,0 g d'acide orthophosphorique cristallisé (acide 100%) sont suspendus dans 170 mL de diisopropyl éther.

La solution d'oléorésine est versée sur la suspension d'acide et le tout est agité pendant une dizaine de minutes, jusqu'à disparition totale de l'acide et obtention d'une solution jaune.

La solution jaune obtenue est alors concentrée à l'évaporateur rotatif. Environ 250 mL de diisopropyl éther sont alors évaporés à l'aide d'un évaporateur rotatif sous une pression de 25 hPa et récupérés pour une utilisation par la suite. Le résidu est repris par 300 mL d'hexane et agité pendant quelques minutes puis laisser à décanter. Une phase huileuse rouge-brique se rassemble au fond du récipient. Cette phase huileuse est alors soutirée et agitée en présence de 100 mL d'hexane. Le mélange est laissé à décanter.

La phase huileuse rouge-brique est reprise par les 250 mL de diisopropyl éther distillés plus haut et traitée comme dans le cas de l'exemple 1 pour donner finalement 39,0 g d'une huile comprenant 100% d'un mélange à au moins 90% de spilanthol vrai accompagné de ses isomères et homologues amides.

### Exemple 4 : concentration du spilanthol contenu dans un extrait de Jambu

25,0 g d'oléorésine de Jambu à 60% de spilanthol vrai sont mis en solution dans 75 mL de benzène et 3,75 mL d'acide sulfurique concentré sont émulsionnés dans 75 mL de benzène sous forte agitation.

L'émulsion est versée sur la solution d'oléorésine sous forte agitation.

Un changement de coloration apparaît et lors de l'arrêt de l'agitation un produit insoluble huileux se dépose au fond du récipient.

Après décantation et lavage au benzène de la phase huileuse le spilanthol est régénéré par neutralisation au carbonate de sodium et extraction au benzène.

La phase benzène est alors séchée, filtrée et concentrée à l'évaporateur rotatif pour fournir 13,5g d'une huile comprenant 100% d'un mélange à au moins 90% de spilanthol vrai accompagné de ses isomères et homologues amides.

### Exemple 5 : concentration du spilanthol contenu dans un extrait de Jambu

25,0 g d'oléorésine de Jambu titrée à 60% de spilanthol vrai sont mis en solution dans 75 mL de diisopropyl éther et 6,5 g d'acide oxalique anhydre sont dissous dans 65 mL du même solvant.

Les deux solutions sont mélangées puis le même traitement que celui pratiqué dans l'exemple 4 est appliqué au mélange.

On obtient alors 14,0 g d'une huile comprenant 100% d'un mélange à au moins 90% de spilanthol vrai accompagné de ses isomères et homologues amides.

### Exemple 6 : concentration du spilanthol contenu dans un extrait de Jambu

25,0 g d'oléorésine de Jambu titré à 60% de spilanthol vrai en solution dans 75 mL de méthylisobutylcétone (MIBK) sont mélangés sous agitation avec une solution de 3,75 mL d'acide sulfurique concentré dans 75 mL de MIBK.

Le milieu s'assombrit mais aucune séparation de phase n'apparait.

Le même traitement que celui appliqué à l'exemple n° 5 est appliqué sur le mélange.

On obtient alors 15,0 g d'une solution huileuse comprenant 100% d'un mélange à au moins 90% de spilanthol vrai accompagné de ses isomères et homologues amides.

### Exemple 7 : concentration du spilanthol contenu dans un extrait de Jambu

60,0 g d'absolue Jambu titré à 40 % de spilanthol vrai sont mis en solution dans 180 mL de Méthyl tert-butyl ether (MTBE) et 9,0 mL d'acide sulfurique concentré sont mis en solution dans 180 mL de MTBE également.

Une réaction se produit au mélange des deux solutions mais aucun produit insoluble n'apparaît comme dans les exemples 1 à 3.

Environ 250 mL de MTBE sont alors évaporés à l'aide d'un évaporateur rotatif Büchi sous une pression de 25 hPa.

Le résidu apparu est alors repris par 360 mL d'hexane sous agitation pendant 5 mn puis laissé à décanter. Une phase huileuse noire se rassemble au fond du récipient. La phase huileuse noire est alors soutirée et agitée en présence de 130 mL d'hexane. Le mélange est laissé à décanter.

La phase huileuse noire est alors reprise par 250 mL d'hexane et traitée comme dans le cas de l'exemple 1 pour donner finalement 24,0 g d'une huile comprenant 100% d'un mélange à au moins 90% de spilanthol vrai accompagné de ses isomères et homologues.

### Exemple 8 : concentration du spilanthol contenu dans un extrait de Jambu à grande échelle

360 g d'oléorésine de Jambu à 60% de spilanthol vrai sont mis en solution dans 1080 mL de MTBE et 54 mL d'acide sulfurique concentré sont mis en solution dans 540 mL de MTBE (cette dernière dissolution est très exothermique, elle est réalisée dans un bain de glace afin que la température reste inférieure à 20°C).

Les deux solutions sont alors mélangées et un produit insoluble sous la forme d'une phase huileuse apparait.

On laisse décanter la phase huileuse sombre pendant au moins dix minutes. La phase huileuse est alors soutirée et lavée par 500 mL de MTBE.

La phase huileuse redécantée est additionnée de 500 mL de MTBE neuf et traitée par environ 1500 mL d'une solution aqueuse de carbonate de sodium à 7,5% pour obtenir un pH final de la solution de 8.

Après décantation et retrait de la phase organique (MTBE), une deuxième extraction est réalisée sur la phase aqueuse avec 250 mL de MTBE.

Dans les deux cas les décantations sont lentes.

Les phases organiques (MTBE) réunies sont lavées par 400 mL de solution de chlorure de sodium (NaCl) à 25%.

Une décantation lente est alors poursuivie pour récupérer une phase organique (MTBE) qui après séchage, filtration et évaporation à sec, permet d'obtenir 208 g d'une huile comprenant 100% d'un mélange à au moins 90% de spilanthol vrai accompagné de ses isomères et homologues amides.

Cet exemple montre que le procédé selon l'invention peut être transposé aisément à une échelle industrielle.

### Exemple 9 : concentration du spilanthol contenu dans un extrait de Jambu à grande échelle

450 g d'oléorésine de Jambu à 60 % de spilanthol vrai sont mis en solution dans 1350 mL (soit 3 vol.) de di-isopropyl éther et 70 mL d'acide sulfurique concentré sont mis en solution dans 1350 mL de di-isopropyl éther en refroidissant au bain de glace. La dissolution de l'acide sulfurique concentré est exothermique, la température passant de +7 à +28°C.

Sous bonne agitation, la solution d'acide est alors versée sur la solution de Jambu. Un précipité jaune, virant au marron se forme.

L'agitation est alors maintenue pendant 2 minutes puis l'ensemble est laissé à décanter pendant au moins 10 minutes.

Une phase huileuse marron se dépose au fond du récipient.

La phase huileuse, assez épaisse, est soutirée et agitée pendant une minute en présence de 675 mL de di-isopropyl éther puis une nouvelle phase de décantation est poursuivie pendant au moins 10 minutes.

La phase huileuse ainsi lavée est reprise par 900 mL de di-isopropyl éther puis est traitée sous bonne agitation avec une solution de 150 g de carbonate de sodium (Na2CO3) dans 750 mL d'eau.

La vérification que tout l'acide a bien été neutralisé (pH autour de 8) est réalisée au papier.

La phase organique est séchée, filtrée et concentrée à l'aide d'un évaporateur rotatif Büchi sous une pression de 25 hPa.

On obtient ainsi 292,3 g (Rdt 65,0%) d'une huile comprenant 100% d'un mélange à au moins 90% de spilanthol vrai accompagné de ses isomères et homologues amides.

### Exemple 10 : concentration du spilanthol contenu dans un extrait de Jambu à échelle industrielle

Dans un erlenmeyer de 2 L, 150 mL d'acide sulfurique à 92% (acide industriel) sont versés par portions, sous agitation, dans 1100 mL (environ 7,5 volumes) de di-isopropyléther, sans dépasser +15°C grâce à un bain réfrigérant.

Pendant ce temps, dans un vaisseau de 5 L, une solution de 750 g de jambu oléorésine à 61% de spilanthol dans 2250 mL (soit 3 volumes) de di-isopropyléther est préparée.

En restant à +15°, la solution d'acide est versée sur la solution de jambu bien agitée. Une huile jaune se sépare, qui vire au marron. L'ensemble est agité encore pendant 3 à 5 mn puis laissé à décanter pendant 10 à 15 mn. Les deux phases sont très propres, l'interface bien nette.

Pendant la décantation, une solution de 300 g de carbonate de sodium dans 1875 mL d'eau est préparée.

L'huile brune est soutirée dans une ampoule et est lavée par 1500 mL de di-isopropyléther. L'ensemble est laissé à décanter. L'huile s'est assombrie.

L'huile lavée est suspendue dans 1500 mL de di-isopropyléther neuf. Sous bonne agitation, la solution de carbonate est ajoutée lentement (15 minutes). L'ensemble est agité encore pendant 5 mn puis laissé à décanter : la phase di-isopropyléther est marron clair, la phase aqueuse est jaune clair, présence de phlegmes. Le pH de la phase aqueuse est vérifié (pH 8 attendu). La phase aqueuse est soutirée et la phase organique est concentrée à l'évaporateur rotatif, sous une pression de 25 hPa, au bain-marie à 60°C. 466,8 g d'une huile comprenant 100% d'un mélange à au moins 90% de spilanthol vrai accompagné de ses isomères et homologues amides sont obtenus.

Une deuxième extraction de la phase aqueuse (750 mL de di-isopropyléther) ne fournit plus que 0,3 g, elle est donc quasiment inutile.

Cet exemple montre que le procédé peut être transposé à échelle industrielle. De plus il montre qu'il est possible de réaliser le procédé selon l'invention avec de l'acide sulfurique à 92%, qui est l'acide sulfurique trouvé dans le commerce, et qui est moins onéreux que l'acide sulfurique concentré par exemple à 98%.

## Revendications

1. Procédé de concentration du spilanthol contenu dans une composition, comprenant :
a. une première étape de mélange d'une composition comprenant du spilanthol avec un solvant inerte aux acides choisi parmi un éther choisi parmi le Diéthyléther, le di-isopropyléther, le Méthyl tert-butyl éther, le Tétrahydrofuranne, le Méthyltétrahydrofuranne, le Dioxane, le Dimethoxyethane, l'Anisole, les éthers de Crown ou encore le Polyethylene glycol, un hydrocarbure choisi parmi les alcanes linéaires ou ramifiés, substitués ou non, comprenant entre 5 et 8 atomes de carbone, ou les cycloalcanes et composés aromatiques substitués ou non, comprenant entre 5 et 8 atomes de carbone ou une cétone choisie parmi l'acétone, la méthyléthylcétone, la méthylisobutylcétone, la pinacolone, la diéthylcétone, la diisopropylcétone, ou encore la 2,4 diméthyl 3 pentanone pour obtenir une solution anhydre comprenant du spilanthol ;
b. une seconde étape de formation d'un produit huileux comprenant un dérivé d'addition du spilanthol par mélange de la solution anhydre obtenue à la première étape avec un acide fort choisi parmi l'acide chlorhydrique, l'acide sulfurique concentré, les acides phosphoriques, l'acide oxalique, l'acide nitrique, l'acide iodhydrique, l'acide bromhydrique, l'acide perchlorique, l'acide chlorique, l'acide permanganique, l'acide manganique, l'acide fluoroantimonique, l'acide magique, l'acide méthanesulfonique, l'acide trifluorométhanesulfonique ou acide triflique, l'acide fluorosulfurique ou acide fluorosulfonique, l'acide naphtalène-1-sulfonique, l'acide naphtalène-1,5-disulfonique, l'acide disulfurique ou oléum, ou encore l'acide paratoluènesulfonique;
c. une troisième étape d'hydrolyse dudit composé d'addition compris dans le produit huileux obtenu à la seconde étape, par mise en présence d'une base choisie parmi le carbonate de sodium, le bicarbonate de sodium, la chaux, l'ammoniaque, la soude, la lithine, la potasse, ou encore la baryte pour obtenir une solution de spilanthol.

2. Procédé selon la revendication 1, **caractérisé en ce que** l'acide fort est rendu anhydre par mélange avec un solvant inerte aux acides choisi parmi le Diéthyléther, le di-isopropyléther, le Méthyl tert-butyl ether, le Méthyltétrahydrofuranne, le benzène, le toluène ou encore la méthylisobutylcétone.

3. Procédé selon l'une quelconque des revendications 1 à 2, **caractérisé en ce que** l'étape de mise en solution anhydre d'une composition comprenant du spilanthol, est réalisée par mélange de ladite composition comprenant du spilanthol avec ledit solvant inerte aux acides pour obtenir une solution finale comprenant ladite composition comprenant du spilanthol en une proportion comprise entre 20 et 50 % poids/volume.

4. Procédé selon l'une quelconque des revendications 2 et 3, **caractérisé en ce que** la solution anhydre d'acide fort est obtenue par mélange dudit acide fort avec un solvant inerte aux acides pour obtenir une solution finale comprenant de l'acide fort en une proportion comprise entre 2 et 15 % volume/volume.

5. Procédé selon l'une quelconque des revendications 1 à 4, **caractérisé en ce qu'**à la troisième étape, la base est ajoutée au produit huileux obtenu à la seconde étape, en un volume représentant de 25% à 75% du volume dudit produit huileux.

6. Procédé selon l'une quelconque des revendications 1 à 5, **caractérisé en ce que** la base utilisée à l'étape d'hydrolyse est en solution, en une proportion comprise entre 5 et 15%.

## Patentansprüche

1. Verfahren zum Konzentrieren des in einer Zusammensetzung enthaltenen Spilanthols, das Folgendes beinhaltet:
a. einen ersten Schritt des Mischens einer Zusammensetzung, die Spilanthol umfasst, mit einem säureinerten Lösungsmittel, ausgewählt aus einem Ether, ausgewählt aus Diethylether, Diisopropylether, Methyl-tert-Butylether, Tetrahydrofuran, Methyltetrahydrofuran, Dioxan, Dimethoxyethan, Anisol, Crown-Ether oder Polyethylenglykol, einem Kohlenwasserstoff, ausgewählt aus linearen oder verzweigten Alkanen, substituiert oder unsubstituiert, umfassend zwischen 5 und 8 Kohlenstoffatome, oder Cycloalkanen und substituierten oder unsubstituierten aromatischen Verbindungen, umfassend zwischen 5 und 8 Kohlenstoffatome, oder einem Keton, ausgewählt aus Aceton, Methylethylketon, Methylisobutylketon, Pinacolon, Diethylketon, Diisopropylketon oder 2,4 Dimethyl-3-pentanon, um eine Spilantholol umfassende wasserfreie Lösung zu erhalten;
b. einen zweiten Schritt des Bildens eines öligen Produkts, das ein Spilanthol-Additionsderivat umfasst, durch Mischen der im ersten Schritt erhaltenen wasserfreien Lösung mit einer starken Säure, ausgewählt aus Salzsäure, konzentrierter Schwefelsäure, Phosphorsäure, Oxalsäure, Salpetersäure, Jodwasserstoffsäure, Bromwasserstoffsäure, Perchlorsäure, Chlorsäure, Permangansäure, Mangansäure, Fluorantimonsäure, magische Säure, Methansulfonsäure, Trifluormethansulfonsäure oder Triflatsäure, Fluorschwefelsäure oder Fluorsulfonsäure, Naphthalin-1-sulfonsäure, Naphthalin-1,5-disulfonsäure, Disulfursäure oder Oleum oder auch Paratoluolsulfonsäure;
c. einen dritten Schritt des Hydrolysierens der Additionsverbindung, die in dem im zweiten Schritt erhaltenen öligen Produkt enthalten ist, durch Zusammenbringen einer Base, ausgewählt aus Natriumcarbonat, Natriumbicarbonat, Kalk, Ammoniak, Soda, Lithin, Kali oder Baryt, um eine Spilanthollösung zu erhalten.

2. Verfahren nach Anspruch 1, **dadurch gekennzeichnet, dass** die starke Säure durch Mischen mit einem säureinerten Lösungsmittel, ausgewählt aus Diethylether, Diisopropylether, Methyl-tert-Butylether, Methyltetrahydrofuran, Benzol, Toluol oder auch Methylisobutylketon, wasserfrei gemacht wird.

3. Verfahren nach einem der Ansprüche 1 bis 2, **dadurch gekennzeichnet, dass** der Schritt des wasserfreien Lösens einer Spilanthol umfassenden Zusammensetzung durch Mischen der Spilanthol umfassenden Zusammensetzung mit dem säureinerten Lösungsmittel durchgeführt wird, um eine Endlösung zu erhalten, die die Zusammensetzung umfasst, die Spilanthol in einer Menge zwischen 20 und 50 Gew./Vol.% umfasst.

4. Verfahren nach einem der Ansprüche 2 und 3, **dadurch gekennzeichnet, dass** die wasserfreie Lösung einer starken Säure durch Mischen der starken Säure mit einem säureinerten Lösungsmittel erhalten wird, um eine Endlösung zu erhalten, die starke Säure in einer Menge zwischen 2 und 15% Volumen/Volumen umfasst.

5. Verfahren nach einem der Ansprüche 1 bis 4, **dadurch gekennzeichnet, dass** im dritten Schritt die Base dem im zweiten Schritt erhaltenen öligen Produkt in einem Volumen von 25% bis 75% des Volumens des öligen Produkts zugesetzt wird.

6. Verfahren nach einem der Ansprüche 1 bis 5, **dadurch gekennzeichnet, dass** die im Hydrolyseschritt verwendete Base in Lösung in einer Menge zwischen 5 und 15% vorliegt.

## Claims

1. Process for the concentration of the spilanthol contained in a composition, comprising:
a. a first step of mixing a composition comprising spilanthol with a solvent that is inert to acids, selected from an ether selected from diethyl ether, diisopropyl ether, methyl tert-butyl ether, tetrahydrofuran, methyl tetrahydrofuran, dioxane, dimethoxyethane, anisole, crown ethers or also polyethylene glycol, a hydrocarbon selected from the linear or branched, substituted or unsubstituted alkanes, comprising between 5 and 8 carbon atoms or the cycloalkanes and substituted or unsubstituted aromatic compounds comprising between 5 and 8 carbon atoms, or a ketone selected from acetone, methyl ethyl ketone, methyl isobutyl ketone, pinacolone, diethyl ketone, diisopropyl ketone, or also 2,4 dimethyl-3-pentanone, in order to obtain an anhydrous solution comprising spilanthol;
b. a second step of forming an oily product comprising an addition derivative of spilanthol by mixing the anhydrous solution obtained in the first step with a strong acid selected from hydrochloric acid, concentrated sulphuric acid, phosphoric acids, oxalic acid, nitric acid, hydriodic acid, hydrobromic acid, perchloric acid, chloric acid, permanganic acid, manganic acid, fluoroantimonic acid, magic acid, methanesulphonic acid, trifluoromethanesulphonic acid or triflic acid, fluorosulphuric acid or fluorosulphonic acid, naphthalene-1-sulphonic acid, naphthalene-1,5-disulphonic acid, disulphuric acid or oleum, or also paratoluenesulphonic acid;
c. a third step of hydrolysis of said addition compound comprised in the oily product obtained in the second step, by bringing it into the presence of a base selected from sodium carbonate, sodium bicarbonate, lime, ammonium hydroxide, soda, lithium hydroxide, potash, or also barium sulphate, in order to obtain a spilanthol solution.

2. Process according to claim 1, **characterized in that** the strong acid is rendered anhydrous by mixing with a solvent that is inert to acids, selected from diethyl ether, diisopropyl ether, methyl tert-butyl ether, methyl tetrahydrofuran, benzene, toluene or also methyl isobutyl ketone.

3. Process according to any one of claims 1 to 2, **characterized in that** the step of putting a composition comprising spilanthol into anhydrous solution is carried out by mixing said composition comprising spilanthol with said solvent that is inert to acids, in order to obtain a final solution comprising said composition comprising spilanthol in a proportion comprised between 20 and 50% weight/volume.

4. Method according to any one of claims 2 and 3, **characterized in that** the anhydrous solution of strong acid is obtained by mixing said strong acid with a solvent that is inert to acids, in order to obtain a final solution comprising strong acid in a proportion comprised between 2 and 15 % volume/volume.

5. Process according to any one of claims 1 to 4, **characterized in that** in the third step, the base is added to the oily product obtained in the second step, in a volume representing from 25% to 75% of the volume of said oily product.

6. Process according to any one of claims 1 to 5, **characterized in that** the base used in the hydrolysis step is in solution, in a proportion comprised between 5 and 15%.
